(19) **Europäisches Patentamt**
**European Patent Office**
**Office européen des brevets**

(11) **EP 2 957 912 A1**

(12) **EUROPEAN PATENT APPLICATION**

(43) Date of publication:
**23.12.2015 Bulletin 2015/52**

(51) Int Cl.:
**G01N 33/543** (2006.01)

(21) Application number: **14172854.3**

(22) Date of filing: **17.06.2014**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**

(71) Applicant: **UNIVERSITY COLLEGE DUBLIN**
**Dublin 4 (IE)**

(72) Inventors:
• **KELLY, Philip**
**Dublin, D5 (IE)**
• **Dawson, Kenneth**
**Dublin, D4 (IE)**

(74) Representative: **Purdy, Hugh Barry**
**PurdyLucey**
**Intellectual Property**
**6-7 Harcourt Terrace**
**D2 Dublin (IE)**

(54) **A method of labelling a target molecule forming part of a corona of molecules on a surface of a nanosized object**

(57) A method of labelling a target molecule forming part of a corona of molecules on a nanosized object is described. The method comprising the steps of incubating the nanosized object with a plurality of probes, in which the plurality of probes comprise small nanoparticles labelled with a recognition motif specific for the target molecule, separating the nanosized object and unbound probe. The invention also provides a method of determining the location or spatial distribution of a molecule forming part of a corona of molecules on the surface of a nanoparticle.

FIG 1.

a

b

**Description**

Introduction

The invention relates to a method of labelling a target molecule forming part of a corona of molecules on a surface of a nanosized object. In particular, the invention relates to a method of determining a spatial characteristic, or an abundance, of at least one target molecule forming part of a corona of molecules on a surface of a nanosized object.

Background to the Invention

It is generally accepted that nanoparticles in a biological milieu form a 'corona' of biomolecules which may be sufficiently long lived, and appropriately organised, to confer key aspects of biological identity to the particle. In current studies the most tightly nanoparticlebound biomolecules (so-called hard corona) are typically stripped from the nanoparticles, and identified *via* mass spectrometry, yielding a macroscopically averaged composition of the biomolecules on the particles. This approach neither addresses their detailed organisation on the nanoparticle surface, nor if the molecules are presented appropriately to activate specific cellular processes. Other methods of analysing the protein corona include X-ray spectroscopy, mass spectrometry and array based studies. X-ray spectroscopy is a time consuming and technically difficult method, which cannot be carried out in high-throughput. Mass spectrometry yields a macroscopically averaged composition of the biomoleulces attached, however, gives no indication about the arrangement or even composition on a single particle basis. Array-based studies are difficult to achieve for nanoparticles; due to the fact that the population is mixed and it is necessary to select for particles which interact, and as such it is not representative of the whole sample.

It is an object of the invention to overcome at least one of the above-referenced problems in reference to analysing biomolecules attached to nanoparticles.

Brief Description of the Invention

Broadly, the invention provides techniques and methods for labelling and subsequently visualising a corona of molecules, generally visualising the biomolecules on the surface of nanoparticles such as are found in a protein corona formed on the surface of a nano-sized object such as a nanoparticle, or bio-functionalised nanomaterials, or a nano-sized biomolecule or hybrid bio-material. The methods of the invention involve labelling specific molecules in the corona (or specific parts of molecules such as epitopes) with probes comprising small nanoparticles with a recognition motif for the target molecule attached. Once labelled, the corona of the nanoparticle can be imaged to determine the spatial characteristics of the corona, for example the location or spatial distribution of target molecule.

This distribution of target molecules is important on several different levels, on a basic level the disposition of these biomolecules across the surface of the nanoparticle yields a fingerprint of the biological identity of the material, which can be used for validation of the material and assessment of quality and batch control. In the case where multiple labels are used with different properties, e.g. different sizes, fluorescent properties, or combinations of a change in density and fluorescence, a multi parametric fingerprint of the nanomaterial can be constructed with varying degrees of detail ranging from single protein distributions to multi correlative patterns which describe the biological identity of the nanosurface. In this instance the latter is of high importance to describe the biological surface of nanomaterials for regulatory or quality control purposes.

Current methods for assessing the quality of nano-drugs or constructs involve measuring the absolute size and zeta potential of the objects which gives information about the physical properties of the nano-constructs, however it does not address or characterise the biological interface in such high detail, a factor which is much more sensitive and subject to change with an alteration of any small detail on the nanoscale e.g. size, surface roughness.

Due to the highly sensitive nature of the biomolecular corona or biological identity of nanoparticles, which in most cases is context specific, it provides us with a unique opportunity to use the distribution of biomolecules across the surface as a characterisation tool assessing in higher detail the reproducibility of nanoparticles, of high import for the regulation of such materials. Moreover the flexibility of this platform allows one to target specific key amino acid residues, or epitopes, which are known to cause specific biological responses, e.g. epitopes which engage cellular receptors facilitating uptake, alert the immune system to potential hazards or trigger other biological effects.

The abundance of the target molecule on the nano-sized object can optionally be quantified by determining a change in physical property e.g. density, once the nanoparticle are labelled, typically by means of density centrifugal sedimentation. In a yet further aspect, the relative abundance of two target molecules forming part of the corona can be determined by determining using contrast in the same physical property or using multiple labels of different types, conferring contrast in multiple parameters e.g. density, fluorescence magnetism etc. Suitable design of the small nanoparticle probe allows specific epitopes of a target protein to be identified and mapped out.

The invention thus provides a method for obtaining information on the location, distribution and orientation of molecules in a nanoparticle corona. The technique is versatile and fast, and provides a quick method for identification of proteins in a protein corona, with the option of further detailed analysis of these proteins. Moreover, by using different sized nanomaterials as the labelling agent, the interaction of biomolecules whilst on a nanoparticle surface can be investigated.

In a first aspect, the invention provides a method of labelling a target molecule forming part of a corona of molecules on a nanosized object, the method comprising the steps of:

- incubating the nanosized object with a plurality of probes, in which the plurality of probes comprise small nanoparticles labelled with a recognition motif specific for the target molecule; and
- Separating the nanosized object and unbound probe.

Generally, the small nanoparticle is configured to act as a contrast agent through one or more parameters such as size, shape, density, fluorescence, charge or magnetism, enabling the visualisation of the small nanoparticle on the surface of the nano-sized object. Various imaging techniques may be employed including electron microscopy, for example TEM (transmission, electron microscopy), SEM (scanning electron microscopy), Helium Ion microscopy. In addition AFM (Atomic force microscopy), STM (scanning tunnelling microscopy), fluorescence imaging and advanced fluorescence techniques such as Förster resonance energy transfer (FRET) and fluorescence lifetime imaging microscopy (FLIM), as well as super resolution fluorescence imaging techniques e.g. Stochastic optical reconstruction microscopy (STORM) and Stimulated emission depletion (STED) can be used.

Typically, the corona is a biomolecular corona, and in which the recognition motif is generally a biological recognition motif.

Preferably, the target molecule is a protein or an epitope of a protein.

Ideally, the biological recognition motif is an antibody, antibody fragment or peptide.

Ideally, the nanosized object is a nanoparticle.

In one embodiment, the method involves labelling a specific part of the target molecule, for example a specific epitope of the target molecule. In this case, the recognition motif is specific for the epitope. Suitable recognition motifs for labelling specific epitopes of target molecules include monoclonal antibodies and peptides, and other biomolecules with defined interactions e.g. lectins and receptors.

Suitably, the method comprises a step of monitoring a change in a physical property of the nanosized object during the incubation step to determine when the nanosized object is saturated with probe, wherein the separating step is generally carried out after the nanosized object has been determined to be saturated with probe. Typically, the physical property is selected from density, light emission, magnetism, or size.

In one embodiment the physical property is density, in which case the small nanoparticle is a heavy probe e.g. gold. In this specification, the term "heavy probe" should be understood to mean a small nanoparticle capable of increasing the net density of the large nanoparticle once bound, typically these particles are dense metals. Similarly if the large nanoparticle of interest is very heavy then a "light probe" may be employed. In this specification the term "light probe" is defined as a small nanoparticle of lower density than the large nanoparticle under analysis, capable of reducing the net density once bound. Suitably, the change is density is determined using centrifugal sedimentation, ideally density centrifugal sedimentation (DCS).

In another embodiment the physical property is light emission, in which the small nanoparticle is a fluorescent or luminescent probe. Examples of such probes include probes in which the small nanoparticle emits light, for example nanocrystals, or small nanoparticles labelled with a light emitting moiety, for example a molecular beacon or a fluorescent or phosphorescent dye. Examples of such dyes include platinum (II) or palladium (II) complexes of porphyrin dyes, organic molecules and luminescent biomolecules. Suitably, the change is light emission is determined using fluorescence/luminescence spectroscopy or an equivalent technique, the details of which will be known to a person skilled in the art.

In another embodiment, the physical property is magnetism, in which case the small nanoparticle has a magnetic character, for example a magnetic core or a magnetic shell. Examples of magnetic probes include superparamagnetic Iron oxide nanoparticles, or other ferromagnetic materials such as are derived from Iron, Cobalt or Nickel. Preferably the change in magnetism is determined by magnetometry or magnetophoresis.

In another embodiment, the physical property is the scattering of the particle in which small nanoparticles alter the way in which large nanoparticles scatter light. This change in scattered light can indicate a change in size, or morphology of the nanoparticle e.g. roughness. Each of the probes discussed previously could be used to change the scattering profile of large nanoparticles. Preferably the change in scattering is measured using Flow Cytometry.

Preferably, the small nanoparticles have a diameter of less than 20 nm, 15nm, 14nm, 13nm, 12nm, 11nm, 10nm, 9mm, 8nm, 7nm, 6nm, or 5nm. Typically, the small nanoparticles have a dimension of at least 0.1nm, 0.5nm, or 1.0nm. The method of measuring the diameter (or dimension) of small nanoparticles (or nanosized objects) is described in National Institute of Standards and Technology (NIST) - NCL Joint Assay Protocol, PCC-7

The invention also provides a method of labelling at least first and second target molecules forming part of a corona of molecules on a nanosized object, the method comprising the steps of:

labelling a first target molecule of a first aliquot of the nanosized object according to a method of any of the invention; and
labelling a second target molecule of a second aliquot of the nanosized object according to a method of the invention.

In another embodiment of this multiple labelling approach labelling the first and subsequent target molecules forming part of a corona of molecules on a nanosized object are labelled using different small nanoparticles this method comprising the steps of:

labelling a first target molecule of a first aliquot of the nanosized object according to a method of any of the invention; and
labelling a second target molecule of a second aliquot of the nanosized object according to a method of the invention whereby the small nanoparticle gives contrast in a different physical property.

The identification and separation of the labelled large nanoparticle is preferably carried out by, so called "multi dimensional" e.g. 2D, 3D techniques, where it is separated in one dimension using contrast in the first physical property and in another dimension with contrast in a second physical property. The number of dimensions is directly proportional to the number of probes conferring different physical properties.

The invention also provides a method of determining a spatial characteristic of at least one target molecule forming part of a corona of molecules on a nanosized object, the method comprising the steps of:

labelling at least one target molecule according to a method of the invention; and generating an image of the nanosized object to detect binding of probe to the nanosized object; and

optionally, analysing the image to determine the spatial characteristic of the target molecule.

The invention also provides a method of determining a spatial characteristic of at least first and second target molecules forming part of a corona of molecules on a nanosized object, the method comprising the steps of:

labelling the at least first and second target molecules according to a method of the invention; and
generating an image of the labelled first aliquot of the nanosized object to detect binding of probe to the nanosized object;
generating an image of the labelled second aliquot of the nanosized object to detect binding of probe to the nanosized object
analysing the images to determine the spatial characteristic of the first and second target ligands.

The invention also provides a method of determining a spatial characteristic of at least first and second target molecules forming part of a corona of molecules on a nanosized object, the method comprising the steps of:

labelling the at least first and second target molecules according to a method of the invention; and
generating an image of the nanosized object to detect binding of probe to the nanosized object; and
analysing the image to determine the spatial characteristic of the first and second target ligands, wherein the probe for the first target molecule and the probe for the second target molecule comprise different physical properties that allow them to be individually determined.

For example, one probe comprises a first small nanoparticle having a first density and the second probe comprises a second small nanoparticle having a different density to the first small nanoparticle. Alternatively, one probe comprises a first small nanoparticle having first magnetic characteristic and the second probe comprises a second small nanoparticle having a different magnetic characteristic to the first small nanoparticle. In another embodiment, one probe comprises a first small nanoparticle having a first density and the second probe comprises a second small nanoparticle having a fluorescence, specific combinations of probes are not limited to the examples listed here.

Typically, the spatial characteristic is selected from the spatial location of the target molecule on the surface of, or attached to the nanosized object, spatial distribution of the target molecules on the surface of the nanosized object, either in relation to other molecules of the same class or, using multiple probes, in relation to molecules of different classes, and orientation of the target molecule on the surface of the nanosized object.

Preferably, the image is generated by microscopy, preferably electron microscopy, for example TEM (transmission, electron microscopy), SEM (scanning electron microscopy), and Helium Ion microscopy. Other methods of generating an image include AFM (Atomic force microscopy), STM (scanning tunnelling microscopy), fluorescence imaging including advanced fluorescence techniques such as Förster resonance energy transfer (FRET) and fluorescence lifetime imaging microscopy (FLIM), as well as super resolution fluorescence imaging techniques e.g. Stochastic optical reconstruction microscopy (STORM) and Stimulated emission depletion (STED).

Generally, when electron microscopy is employed, the small nanoparticle is a heavy probe, for example gold.

Typically, when fluorescence imaging is employed, the probe has a fluorescence or luminescence characteristic, for example a nanocrystal or a small nanoparticle labelled with a fluorescent probe.

Typically, when magnetic imaging is employed, the probe is at least partially magnetic.

Preferably, the first target molecule is a first a protein, and the second target molecule is a second protein.

In another embodiment, the first target molecule is a first epitope of a protein, and the second target molecule is a second epitope of the same protein.

In another embodiment, the first target molecule is a first epitope of a protein, and the second target molecule is a different epitope of a different protein.

The invention also provides a method of determining the abundance of at least one target molecule forming part of a corona of molecules on a surface of a nanosized object, the method comprising the steps of:

labelling the at least one target molecule according to a method of the invention;
determining a change in physical property of the labelled nanosized object; and
correlating this change in physical property of the labelled nanosized object with the abundance of the at least one target molecule.

The invention also provides a method of determining relative abundance of at least first and second target molecules forming part of a corona of molecules on a surface of a nanosized object, the method comprising the steps of:

labelling the at least first and second target molecules according to a method of the invention;
determining a change in physical property of the labelled nanosized object in the first aliquot;
determining the change in physical property of the labelled nanosized object in the second aliquot; and
correlating the change in physical property of the labelled nanosized object in the first aliquot and the change in physical property of the labelled nanosized object in the second aliquot to provide the relative abundance of the first and second target molecules.

Typically, the physical property is selected from density, light emission, charge, magnetism, or size. Examples of methods to determine the change in these physical properties, and suitable probes to employ, are described in more detail above.

The invention also relates to a method of mapping out the distribution of epitopes on a nanoparticle surface comprising the steps of determining the determining the spatial location of each of the epitopes on the nanoparticle surface according to a method of the invention.

Definitions

In this specification, the term "nanosized object" should be understood to mean objects having a size range of 10-5000nm, typically 10-1000nm, 200-1000nm, 300-1000nm, 400-1000nm or 500nm to 1000nm, including nanoparticles and sub-micron sized biological molecules and clusters including virus, cellular organelles (for example endosomes, exosomes, mitochondria), and biological complexes (for example lipoprotein molecules). Examples of nanoparticles include polymer nanoparticles such as polystyrene, inorganic nanoparticles, for example silica, gold or silver, composite and biocomposite nanoparticles including hybrid materials, combining a biological and synthetic element, and biological objects on the nanoscale eg. liposomes and organelles.

In this specification, the term "small nanoparticle" should be understood to mean nanoparticles that have a dimension not greater than 20% of the dimension of the nanosized object to be labelled, and having a size range of 0.1 to 20nm. Examples of suitable small nanoparticles are colloidal gold, quantum dots, superparamagnetic nanoparticles, nanoclusters and nanostructures.. Generally, the small nanoparticle will have a dimension that is not greater than 10% or 5% of the dimension of the nano-sized object.

In this specification, the term "corona of molecules" should be understood to mean the molecules that adhere to the surface of a nano-sized object. Typically, the molecules are biomolecules. Generally, the molecules are protein in which case the corona is referred to as a protein corona. However, in certain circumstances the corona of molecules may include other biomolecules (for example monomeric and polymeric sugar molecules, and nucleic acids), chemical molecules, or a mixture of biomolecule and chemical molecules. Generally, the corona of molecules will comprise a plurality of different molecules, for example a multiplicity of proteins. For example, when a nanoparticle is located in a biological fluid comprising a milieu of biomolecules, a corona of the biomolecules comprising predominantly protein molecules will form on the surface.

In this specification, the term "target molecule" should be understood to mean a specific biomolecule or chemical molecule that forms part of the corona of molecules on the curved surface of the nano-sized object. Examples of target molecules include specific proteins, peptides, polypeptides, sugars, nucleic acids, and chemical molecules, or a conjugate of any of these. Preferably, the target molecule a a protein. In one embodiment, the methods of the invention comprise labelling a specific part of a target molecule, for example a specific epitope of the target molecule.

In this specification, the term "recognition motif specific for the target molecule" refers to a ligand forming part of the probe that is capable of specific binding with the target molecule. The recognition motif may be a biomolecule or a chemical molecule. Preferably, the recognition motif is a biological recognition motif, for example an antibody or antibody fragment, protein, peptide, or aptamer.

In the specification, the term "epitope" should be understood to mean an exposed part of a protein that is recognised by an antibody or antibody fragment. Typically, a protein will have a number of different epitopes.

The method of the invention comprises separating the nano-sized objects from unbound probe. Methods for separation will be apparent to those skilled in the art, and generally include washing the nano-sized objects to remove the excess probe.

In this specification, the term "imaging" or "generating an image" should be understood to mean any technique in which the surface of the nanosized object can be visualised such that bound probe can be differentiated from the surface of the nanosized object (i.e. the probe acts as an imaging contrast agent. Suitably imaging techniques include, but are not limited to , electron microscopy for example TEM (transmission, electron microscopy), SEM (scanning electron microscopy), and Helium Ion microscopy. Other methods of generating an image include AFM (Atomic force microscopy), STM (scanning tunnelling microscopy), fluorescence imaging including advanced fluorescence techniques such as Förster resonance energy transfer (FRET) and fluorescence lifetime imaging microscopy (FLIM), as well as super resolution fluorescence imaging techniques e.g. Stochastic optical reconstruction microscopy (STORM) and Stimulated emission depletion (STED)..

The small nanoparticles are labelled with the recognition motif using techniques that are known to the person skilled in the art, for example by covalent attachment through the use of linkers or short molecules to couple the small nanoparticle and the recognition motif, or through modification of the small nanoparticle, or recognition motif to allow specific attachment, direct attachment to the surface of the nanoparticle, adsorbing the recognition motif. Examples of modification include modifying the chemical groups of the small nanoparticle to facilitate specific chemical attachment, modifying the surface of the small nanoparticle with a biological molecule e.g. streptavidin, protein G, Protein A to facilitate attachment of the recognition motif.

Brief Description of the Figures

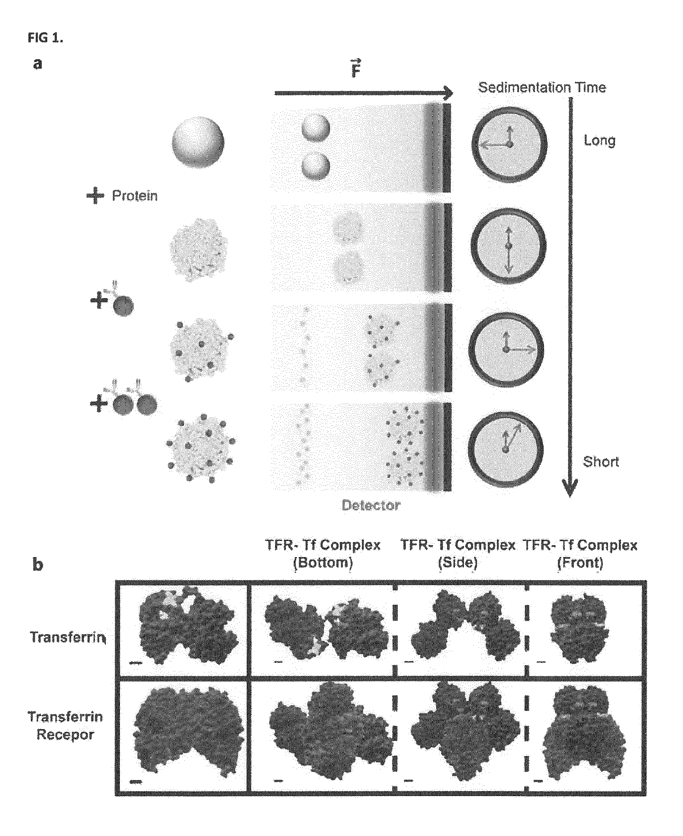

**Figure 1.** *Schematic representation of Immunogold labelling methodology, 3D depiction of relevant Transferrin epitopes.* The addition of proteins or immunogold increases the net density of the polystyrene nanoparticles and reduces the sedimentation time of these particles in a disc centrifuge, causing them reach the detector faster (a). The diameter reported by the instrument is then incorrect as it assumes the density of the unmodified material, this change in apparent diameter can be measured and related to the amount of substance attached (SD1). 3D structure of transferrin (blue) TfR (green) and receptor bound to two Transferrin molecules (blue and red) (b). The epitope implicated in TfR binding (Leu122-Asp125) is highlighted in yellow, whilst the epitope recognised by the monoclonal antibody (Pro142-Pro145) is highlighted in green. It can be observed that spatially these epitopes are close together and are both orientated on the receptor binding side of the protein.

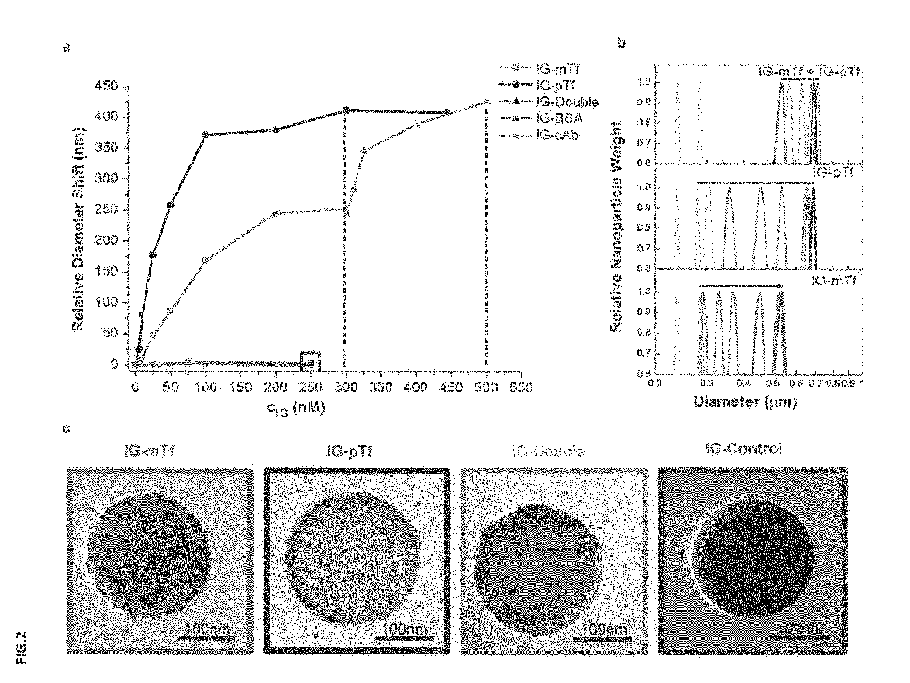

**Figure 2.** *Immunogold labelling and epitope mapping of Transferrin coated polystyrene.* DCS measurements for Transferrin coated polystyrene particles (a) incubated with increasing concentrations of IG-mTf (red), IG-pTf (black), Transferrin coated polystyrene particles saturated with IG-mTf and subsequently titrated with IG-pTf (green), Transferrin coated polystyrene titrated with gold nanoparticles with a control antibody and with no antibody (purple and blue) showing no significant shift, indicating no interaction. It is clear from this graph that both IG-mTf and IG-PTf saturate albeit at different final shifts, indicating a lower abundance of epitopes recognised by IG-mTf. Moreover, after saturating with IG-mTf it is possible to reach the same relative diameter shift as seen by IG-pTf. The DCS distributions are shown (b) for the top 40% of the peak. Representative TEM images of the saturation point for each condition (c) illustrate the labeling of the Tf particles using the different immunogold labels IG-mTf and IG-p-Tf, the control and the double titration.

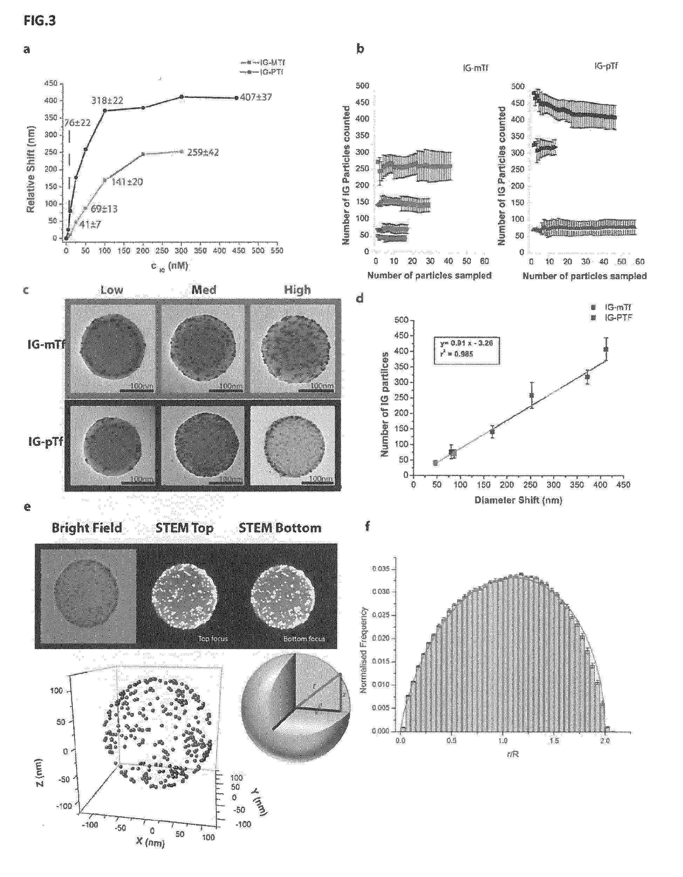

Figure 3. *Counting the number of epitopes exposed on the surface of nanoparticles.* The number of IG particles attached was measured for several points on the titration curve for both IG-mTf and IG-pTf (a). The mean value of IG labels per PS particle converges after a few 10's of images (b) indicating a reasonable estimate of the mean number of epitopes labelled for each conditon. Representative electron microscopy images (c) for low, medium and high points on the titration curve are shown, highlighting that more epitopes are labelled for IG-pTf. Using the different conditions tested a calibration curve was constructed (d) showing that the shift observed in the DCS is directly proportional to the number of IG particles attached, whist this is true in this instance a more general description of the change in apparent diameter can be found in SD1. Using STEM imaging it was possible to gain z contrast and thus identify the position of each IG sphere on the surface of a PS nanoparticle (e), this enabled a 3D reconstruction of a single labelled PS nanoparticle to be obtained. Radial distribution corroaltions were used to determine if there were any long-range patterning or organsation of proteins on the nanoparticle surface (f). The experimental derived corrolations (bar chart) agree with a completely random organisation, which confirms a stochastic adsorption process for this system.

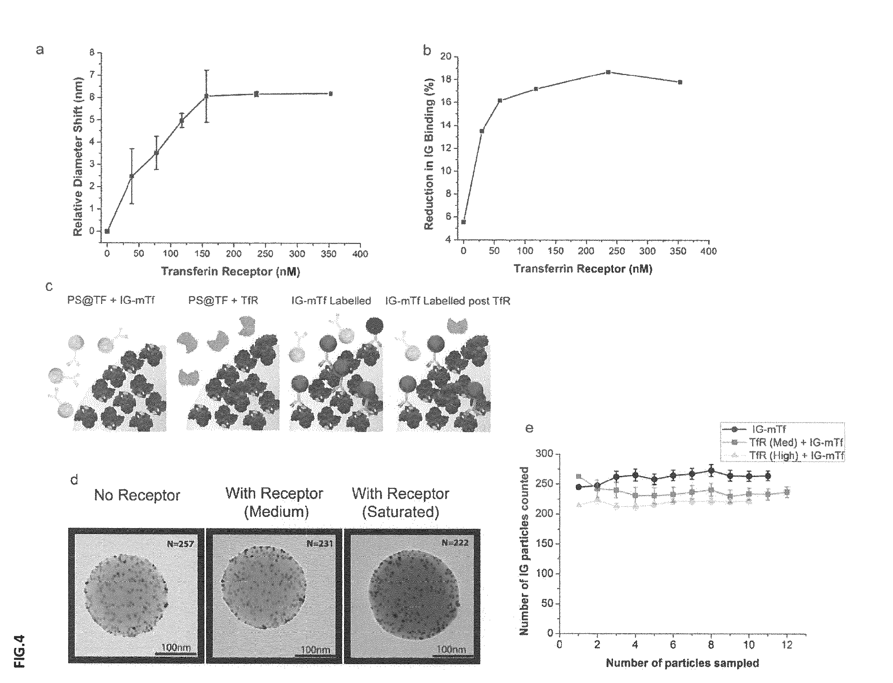

Figure 4. *Interaction of Transferrin coated polystyrene with Transferrin Receptor.* Using DCS it was possible to investigate the interaction of soluble homodimeric TfR with Transferrin coated polystyrene nanoparticles. The receptor binding curve (a) shows that the receptor is capable of interacting with the transferrin molecules on these particles. If the Transferrin coated polystyrene nanopartcles are incubated with TfR and subseqently labelled with IG-mTf there is a reduction in the number of IG particles bound as determined by DCS (b) this reduction saturates at the same TfR concentration as the binding curve for TfR. A schematic representation of what is occuring is shown (c) where nanoparticles that are pre-incubated with TfR demonstrate a reduction in the number of IG particles in comparison with their counterparts free from TfR, two colours of IG are used to distinguish the unbound from the bound labels. Electron microscopy images (d) were taken of samples blocked with medium and high amounts of TfR prior to IG-mTf labelling. The mean number of IG particles counted for each of these blocked conditions as a function of number of particles counted (e) shows the averages for these samples are robust, in addition a clear difference can be observed between the sample which is blocked with a high amount of TfR (light grey) and the particles which have not been bloked (black).

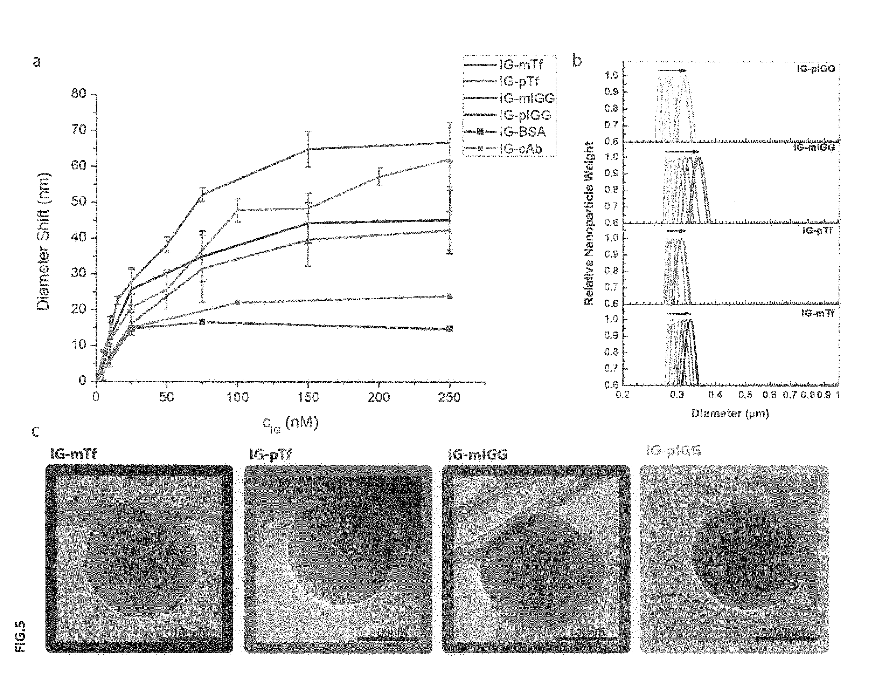

**Fig 5.** *Immunogold labeling of the nanoparticle biomolecular corona.* DCS measurements for PS@HC (a) incubated with increasing concentrations of IG-mTf (black), IG-pTf red), IG-mIgG (blue), IG-pIgG (green) and controls of IG-BSA and IG-Control Antibody (navy and purple). The DCS distributions are shown (**b**) for the top 40% of the peak. More IG particles bind for IgG with respect to Transferrin, in line with the relative abundance of these proteins determine by mass spectrometry. Representative TEM images (**c**) are shown for the saturation point of each condition.

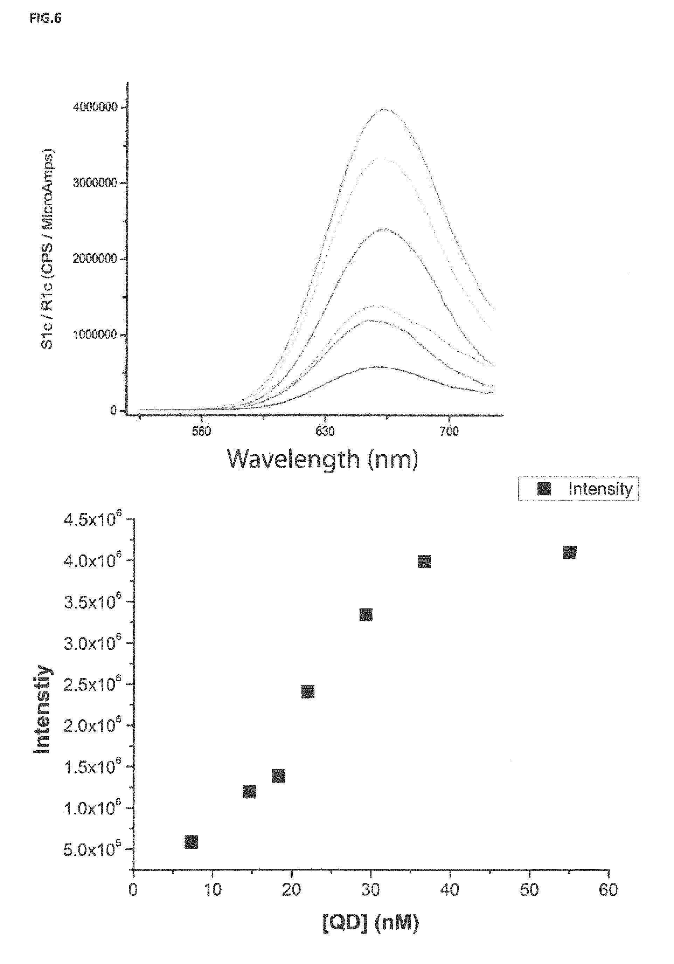

**Fig. 6:** *Luminescent intensity of PS@TF QD-labelled nanoparticles after excess labelling agent is washed away.* The luminescent intensity saturates indicating that all the available epitopes have been labelled.

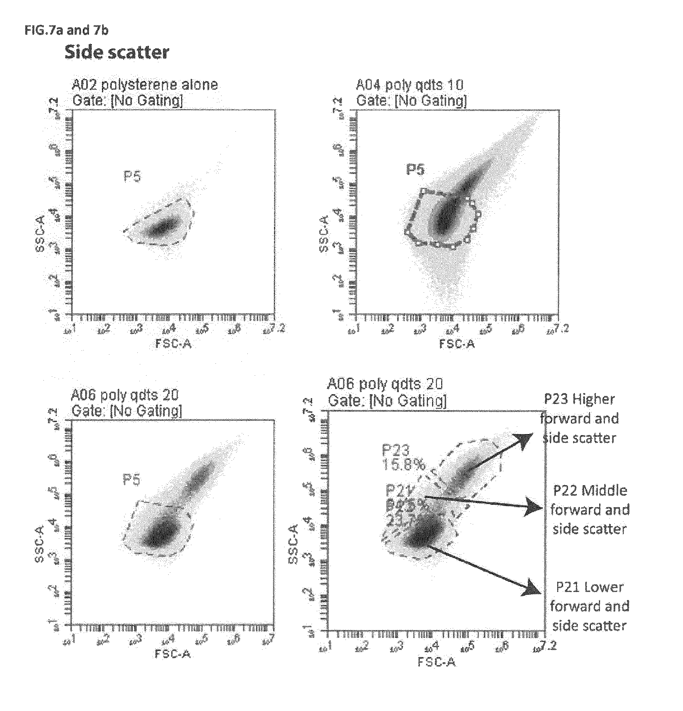

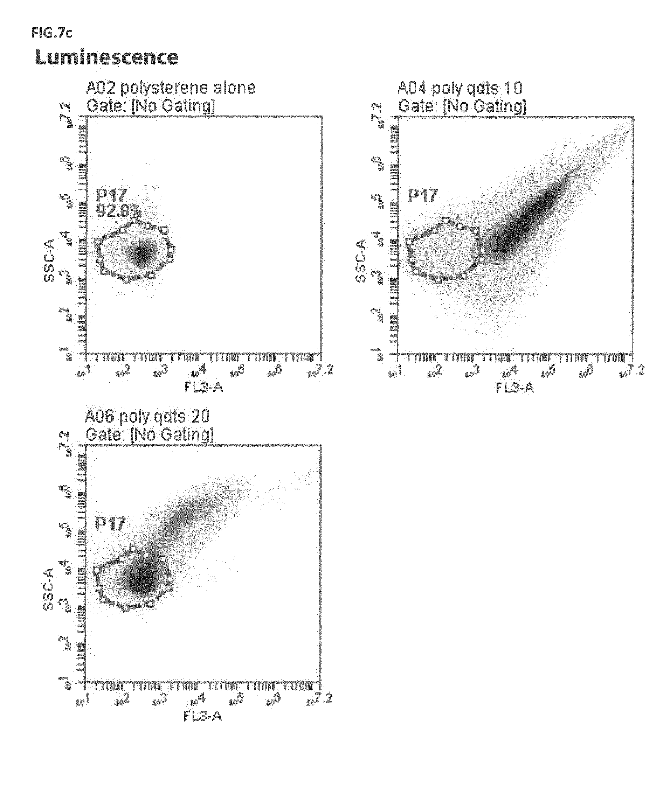

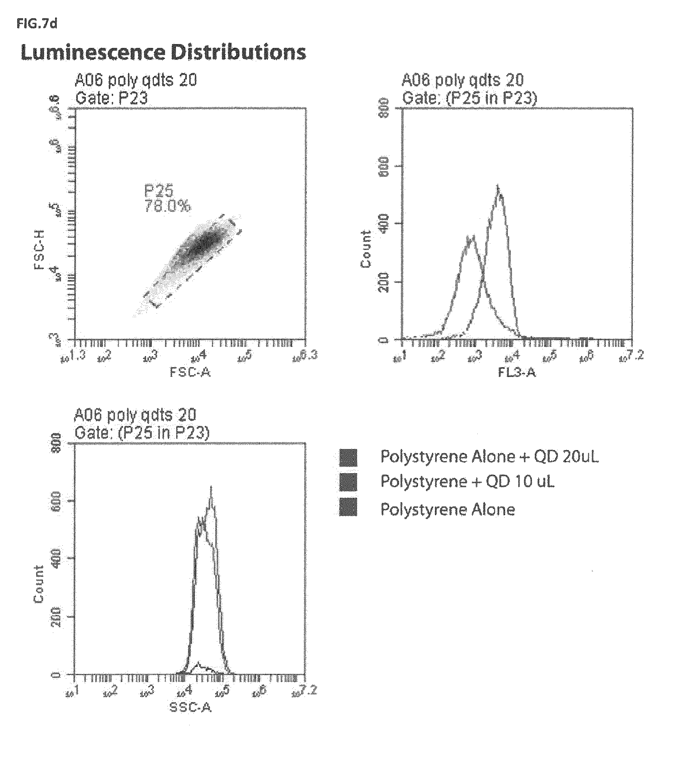

**Fig. 7:** *Flow Cytometry of QD-antibody PS@Tf.* (a) Side scatter vs forward scatter for polystyrene nanoparticles coated in transferrin with no QD-ab added, a medium amount and saturated with QD's. (b) Expanded view to demonstrate the detection of subpopulations and thus the sorting capabilities. (c) Side scatter vs luminescence intensity for the same samples as analysed in (a). (d) Luminescent distributions across the sample

Detailed Description of the Invention

Materials and Methods

*Small Nanoparticle synthesis and characterisation*

Small gold nanoparticles were produced according to the Slot-Geuze method. [Slot, J. W. & Geuze, H. J. A new method of preparing gold probes for multiple-labeling cytochemistry. European journal of cell biology 38, 87-93 (1985).]
The particles were characterised using UV-visible spectroscopy, Differential Centrifugal Sedimentation and Transmission Electron Microscopy.

*Differential Centrifugal Sedimentation*

All DCS measurements of gold nanoparticles were carried out using an 8-24% sucrose density gradient using PBS buffer as the aqueous component, with a disc speed set to 24000 rpm while monitoring the 0-100 nm range. Each particle size measurement was calibrated using a PVC standard of nominal diameter 476 nm. No particle aggregation was observed in the investigated size range. Polystyrene samples were analysed using a 2-8% sucrose density gradient prepared in PBS (0.137 M, pH 7.4) at a disc speed of 24000 rpm. Each measurement was calibrated with a polystyrene standard of nominal diameter 497 nm.

*Transferrin Coated Polystyrene Titrations*

The kinetics of IG binding was investigated by DCS showing that equilibrium is reached after 30 min. For immunogold titrations with Transferrin coated polystyrene nanoparticles the PS concentration was fixed at 50 $\mu$g/mL, the concentrations of immunogold were varied between 1-300 nM, samples were incubated for 1 h at room temperature prior to DCS analysis. The samples were injected onto the disc without any further processing as sedimentation through the disc separates the unbound gold from the PS (Fig. 1). The change in the apparent diameter of the particles is calculated using Equation 1. Where $d_i$ is the diameter of the protein coated particles and $d_s$ is the apparent diameter of the sample post gold labelling.

$$Relative\ Shift =\ d_s - d_i \quad (1)$$

For the double titration, the Transferrin coated polystyrene was saturated with IG-mTf and washed free of excess gold by three cycles of centrifugation at 20000 x g for 10 min. These particles were subsequently titrated with IG-pTf. Analysis of the washed samples indicated that negligible gold was removed from the surface during the washing procedure (Fig.2a). Two controls were used to check the level of nonspecific binding of gold nanoparticles. The first was IG with no antibodies in which the surface was blocked with BSA (IG-BSA). The second control used an antibody raised against R-phycoetherin (IG-cAb), carefully chosen to have minimal cross-reactivity with human plasma proteins. Both controls showed minimal binding at the same concentrations where IG-mTf and IG-pTf were already saturated confirming that the shifts are due to specific binding of the antibodies to their target epitopes (Fig.2a).

*Polystyrene Corona (PS@HC) Titrations*

Carboxylated PS nanoparticles of nominal diameter 200 nm were incubated for 1 h at room temperature in 80% v/v plasma at a nanoparticle concentration of 1 mg/mL. These nanoparticles complete with corona (PS@HC) were washed six times by centrifuging 250 $\mu$L at 20000 x g for 10 min and re-suspending in PBS (137mM, pH 7.4, with 1mM EDTA). The biomolecular corona was analysed by SDS-PAGE. Immunogold labelling was carried out by incubating PS @HC with different immunogold concentrations 1-300 nM overnight at 4°C prior to analysis using DCS. These incubation conditions were chosen to reduce nonspecific binding of IG labels. The results shown are the average of three measurements carried out on independently prepared samples (Fig.5).

The same IG controls that were used for the Transferrin coated polystyrene nanoparticles were applied to the corona particles. A small shift was observed for both these controls when incubated with PS@HC demonstrating a minor level of nonspecific interaction (Fig.5a). Human plasma was depleted of Transferrin following a previously reported method (Salvati, A. et al. Transferrin-functionalized nanoparticles lose their targeting capabilities when a biomolecule corona adsorbs on the surface. Nature nanotechnology 8, 137-143 (2013)). This was used to form a corona on PS-COOH nanoparticles and labelled with IG-mTf, IG-pTf, IG-BSA and IG-cAb, the results show a reduction in IG-mTF binding; however an element of nonspecific binding was observed for IG-pTf, potentially due to cross-reactivity or detection of homologous epitopes in other proteins. This was later confirmed using immunoblots, both for proteins in solution and adsorbed onto nanoparticles.

*Investigation of Transferrin Receptor (TfR) Interactions*

Transferrin coated polystyrene (50 $\mu$g/mL) was incubated with increasing amounts of soluble TfR ca. 1-300 nM for 1 h at room temperature. The apparent diameter of these particles was measured by DCS and compared with particles in the absence of any receptor. The results shown are representative of duplicate measurements (Fig.4a), a single data point at high receptor concentrations ca. 350 nM was measured to ensure saturation had been reached. Transferrin coated polystyrene nanoparticles were incubated with different concentrations of TfR and subsequently labelled using IG-mTf at a concentration corresponding to saturation. The samples were analysed by DCS and the percentage reduction in IG binding was calculated using Equation (2). Where $d_{block}$ is the apparent diameter of the sample after blocking of the surface with TfR and labelling with IG-mTf, and $d_{sat}$ is the diameter for the particles saturated with IG-mTf (Fig.3b). Samples corresponding to medium (50 nM) and high (200 nM) TfR concentrations were imaged by electron microscopy and the number if IG particles attached counted (Fig.4c).

$$\%reduction = 1 - \left(\frac{d_{block}}{d_{sat}}\right) * 100 \quad (2)$$

*Scanning Transmission Electron Microscopy (STEM) and 3D Reconstruction*

STEM imaging was used to generate a 3D reconstruction of a Transferrin coated polystyrene saturated with IG-mTf (Fig.3e). A schematic designed to show how particles distributed around a sphere can be viewed in STEM mode. In particular, the difference in focus between particles on the top and the bottom can be achieved through careful analysis of each particle on the surface by comparing bright field, and the two STEM modes. It should be noted that identification around the circumference of the sphere is the most problematic.

*Protein Structures*

Protein structures were obtained from the protein databank www.rcsb.org (PDB ID: 1SUV) (Scheme1).[16] These structures were visualised and labelled using UCSF Chimera (Pettersen, E. F. et al. UCSF chimera - A visualization system for exploratory research and analysis. J Comput Chem 25, 1605-1612 (2004)).

*Quantum Dots for use as labelling agents*

Figs 6 and 7 demonstrate the feasibility of using quantum dot particles conjugated to antibodies to label the epitopes of protiens on the surface of a nanoparticle. Using these luminescent probes different techniques can be employed to examine the extent of labelling e.g. luminescent spectroscopy, or the distribution of luminescent intensitites across the sample flow cytometry. Figure 6 shows a titration curve in which polystyrene nanoparticles of nominal diameter 200nm are incubated with quantum dots conjugated with an antibody which recognises transferrin. The samples are washed to remove any unbound quantum dots and thus the fluorescent intensity is proportional to the number of quantum dots attached to the polystyrene nanoparticles.

The luminescent properties of quantum dots allow for analysis by different techniques, to illustrate this flow cytomety was used to distinguish between nanoparticles pre and post labeling with the same QD-Ab labels as were used for figure 6. Figure 7(a) shows the change in the forward (xaxis) and side scatter (y axis) of PS@Tf nanoparticles post labeling with QD-Ab's. this change is the scattering arises due to the increase in size of the object and change in optical properties. Figure 7(b) Highlights that it is possible to separate different populations using flow cytometry and these populations can be sorted to obtain distinct samples if neccisary. Figure 7(c) shows that in additon to forward and side scatter the luminescent intensity can be used to show follow a change in the sample post labelling with QD-Ab's. Figure 7(d) shows the luminescent distributions for each of the samples demonstrating a quick method to discribe the distribution of labeled proteins across a larege number of nanoparticles.

The methods of the invention may be employed to map out the proteins fon a nanosized object such as a nanoparticle, to determine the location and spatial distribution of the protein. The methods may also be employed to map out specific epitopes of biomolecules on a nanoparticle, providing information on the orientation of the proteins that contain the specific epitope. The methods of the invention may also be employed to determine the abundance of specific protein, or epitope, on a nanoparticle, and to determine relative abundance of a plurality of different proteins, different epitopes, including different epitopes from the same protein.

The invention is not limited to the embodiments hereinbefore described which may be varied in construction, detail and process step without departing from the spirit of the invention.

**Claims**

1. A method of labelling a target molecule forming part of a corona of molecules on a nanosized object, the method comprising the steps of:

   incubating the nanosized object with a plurality of probes, in which each probe comprises a small nanoparticle labelled with a recognition motif specific for the target molecule; and
   separating the nanosized object and unbound probe.

2. A method as claimed in Claim 1 in which the target molecule is a protein, and the corona is a biomolecular corona.

3. A method as claimed in Claim 2 in which the recognition motif is an antibody or antibody fragment that is specific for an epitope of the target molecule.

4. A method as claimed in any preceding Claim in which the nanosized object is a nanoparticle having a diameter of less than 1000nm and in which the small nanoparticle has a diameter of less than 10nm.

5. A method as claimed in any preceding Claim including a step of monitoring a change in a physical property of the nanosized object during the incubation step to determine when the nanosized object is saturated with probe, wherein the separating step is carried out after the nanosized object has been determined to be saturated with probe.

6. A method as claimed in any preceding claim where the physical property is selected from density, fluorescence, magnetism, charge and size.

7. A method as claimed in Claim 6 in which the physical property is density, and in which the density is determined by differential centrifugal sedimentation (DCS).

8. A method of labelling at least first and second target molecules forming part of a corona of molecules on a nanosized object, the method comprising the steps of:

labelling a first target molecule of a first aliquot of the nanosized object according to a method of any of Claims 1 to 7; and
labelling a second target molecule of a second aliquot of the nanosized object according to a method of any of Claims 1 to 7.

9. A method according to Claim 8 in which the first target molecule is labelled with a first probe having a first physical property and the second target molecule is labelled with a second probe having a second physical property that is different to the first physical property, and wherein one or both of the first and second physical properties are used to separate and/or identify the nanosized object.

10. A method of determining a spatial characteristic of at least one target molecule forming part of a corona of molecules on a nanosized object, the method comprising the steps of:

labelling the at least one target molecule according to a method of any of Claims 1 to 7; and
generating an image of the nanosized object to detect binding of probe to the nanosized object; and
analysing the image to determine the spatial characteristic of the target molecule.

11. A method of determining a spatial characteristic of at least first and second target molecules forming part of a corona of molecules on a nanosized object, the method comprising the steps of:

labelling the at least first and second target molecules according to a method of Claim 8 or 9; and
generating an image of the labelled first aliquot of the nanosized object to detect binding of probe to the nanosized object;
generating an image of the labelled second aliquot of the nanosized object to detect binding of probe to the nanosized object; and
analysing the images to determine the spatial characteristic of the first and second target ligands.

12. A method as claimed in Claim 11 in which the spatial characteristic is selected from spatial location of the target molecule on the surface of the nanosized object, spatial distribution of the target molecule on the surface of the nanosized object, and orientation of the target molecule on the surface of the nanosized object.

13. A method as claimed in Claim 8, 9, 11 or 12, in which the first target molecule is a first epitope of a protein, and the second target molecule is a second epitope of the same protein, or in which the first target molecule is a first protein, and the second target molecule is a second protein.

14. A method of determining the abundance of at least one target molecule forming part of a corona of molecules on a surface of a nanosized object, the method comprising the steps of:

labelling the at least one target molecule according to a method of any of Claims 1 to 7;
determining the change in physical property of the labelled nanosized object; and
correlating this change in physical property of the labelled nanosized object with the abundance of the at least one target molecule.

15. A method of determining relative abundance of at least first and second target molecules forming part of a corona of molecules on a surface of a nanosized object, the method comprising the steps of:

labelling the at least first and second target molecules according to a method of Claim 9; and
determining the change in physical property of the labelled nanosized object in the first aliquot;
determining this change in physical property of the labelled nanosized object in the second aliquot; and
correlating the change in physical property of the labelled nanosized object in the first aliquot and the change in physical property of the labelled nanosized object in the second aliquot to provide the relative abundance of the first and second target molecules.

FIG 1.

a

b

**FIG.2**

**FIG.3**

FIG.4

**FIG.5**

FIG.6

FIG.7a and 7b

## Side scatter

FIG.7c

# Luminescence

FIG.7d

# Luminescence Distributions

Europäisches
Patentamt

European
Patent Office

Office européen
des brevets

# EUROPEAN SEARCH REPORT

**Application Number**

EP 14 17 2854

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | MARA CANOVI ET AL: "Applications of Surface Plasmon Resonance (SPR) for the Characterization of Nanoparticles Developed for Biomedical Purposes", SENSORS, vol. 12, no. 12, 27 December 2012 (2012-12-27), pages 16420-16432, XP055153960, ISSN: 1424-8220, DOI: 10.3390/s121216420 * pg 16421-16428. * | 1-9,13 | INV. G01N33/543 |
| X | WO 2010/097785 A1 (UNIV DUBLIN [IE]; DAWSON KENNETH [IE]; LYNCH LSEULT [IE]; LUNDQVIST MA) 2 September 2010 (2010-09-02) * pg 4, third par-pg 5, first par; pg 9, last par-pg 10, scond par; pg 15, second par; pg 23, fourth par-pg 24, first par; pg 49, second par;cl 6-12, 32-33; fig 18, fig 20, fig 21, fig 22. * | 1-15 | |
| X | WO 2011/088128 A2 (UNIV CENTRAL FLORIDA RES FOUND [US]; HUO QUN [US]) 21 July 2011 (2011-07-21) * par 00023, 00028, 00033, 00039-00041; cl 1-3, 5, 10-11, 17-20, 23, 26-27. * | 1-3,5-9, 13 | TECHNICAL FIELDS SEARCHED (IPC) G01N |

The present search report has been drawn up for all claims

| Place of search | Date of completion of the search | Examiner |
|---|---|---|
| Munich | 21 November 2014 | Motrescu-Hateley, E |

CATEGORY OF CITED DOCUMENTS

X : particularly relevant if taken alone
Y : particularly relevant if combined with another
document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

**ANNEX TO THE EUROPEAN SEARCH REPORT
ON EUROPEAN PATENT APPLICATION NO.**

EP 14 17 2854

This annex lists the patent family members relating to the patent documents cited in the above-mentioned European search report.
The members are as contained in the European Patent Office EDP file on
The European Patent Office is in no way liable for these particulars which are merely given for the purpose of information.

21-11-2014

| Patent document cited in search report | Publication date | Patent family member(s) | | Publication date |
|---|---|---|---|---|
| WO 2010097785 A1 | 02-09-2010 | EP | 2401619 A1 | 04-01-2012 |
| | | US | 2012046184 A1 | 23-02-2012 |
| | | WO | 2010097785 A1 | 02-09-2010 |
| WO 2011088128 A2 | 21-07-2011 | CA | 2787201 A1 | 21-07-2011 |
| | | CN | 102812358 A | 05-12-2012 |
| | | EP | 2524219 A2 | 21-11-2012 |
| | | US | 2013052661 A1 | 28-02-2013 |
| | | WO | 2011088128 A2 | 21-07-2011 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Non-patent literature cited in the description**

- **SLOT, J. W. ; GEUZE, H. J.** A new method of preparing gold probes for multiple-labeling cytochemistry. *European journal of cell biology,* 1985, vol. 38, 87-93

- **SALVATI, A et al.** Transferrin-functionalized nanoparticles lose their targeting capabilities when a biomolecule corona adsorbs on the surface. *Nature nanotechnology,* 2013, vol. 8, 137-143
- **PETTERSEN, E. F. et al.** UCSF chimera - A visualization system for exploratory research and analysis. *J Comput Chem,* 2004, vol. 25, 1605-1612